# EUROPEAN PATENT SPECIFICATION

(11) **EP 0 502 714 B1**
(45) Date of publication and mention of the grant of the patent: **08.11.1995**
(21) Application number: 92301863.4
(22) Date of filing: 04.03.1992
(51) Int. Cl.: A61M 25/06, A61L 29/00

(54) **Use of surfactants to improve intravenous catheter flashback**
Verwendung von oberflächenaktiven Mitteln zur Verbesserung des Rückflusses von Venenkathetern
Utilisation d'agents tensio-actifs pour améliorer la reflux sanguin des cathéters intraveineux

(30) Priority: 04.03.1991 US 664079
(43) Date of publication of application: 09.09.1992
(73) Proprietor: CRITIKON, INC., Tampa, Florida 33631-3800 (US)
(72) Inventor: van Heugten, Anthony Y., Tampa, Florida 33635 (US); Cannon, Julian E., Brandon, Florida 33510 (US)
(74) Representative: Fisher, Adrian John

(56) References cited:
- US-A- 3 861 396
- US-A- 4 762 516
- US-A- 4 861 617

## Description

### Field of the Invention

This invention relates generally to intravenous catheters. In particular, this invention relates to intravenous catheters which cover the cannula tip after use to prevent accidental injury from used needles. Specifically, this invention relates to the improvement of flashback within intravenous catheters to improve their methods of use.

### Background of the Invention

Intravenous catheters for the infusion of fluids into the peripheral veins of a patient are one of the most common devices used in I.V. therapy. I.V. catheters may be produced in two general forms: through-the-needle catheters, in which a catheter is threaded through the needle cannula and into the vein of a pateient, and over-the-needle catheters, in which the needle and concentric outer catheter are inserted into the vein and the needle is withdrawn through the emplaced catheter.

A typical over-the-needle I.V. catheter requires the user to remove and then dipose of a contaminated needle after the needle tip and catheter are properly located in the vein of the patient. Once the needle is withdrawn from the catheter, the user's immediate priorities are infusion set connection and site preparation, including the taping of the catheter to the patient. Because of the urgency of these procedures, the needle is normally just dropped conveniently nearby and then retrieved later. Since the needle at this time is exposed and located close to where the user is completing work with the catheter, accidental self-inflicted needle injuries are not uncommon. For reasons of the desirability of protecting the user from exposure to hepatitis and AIDS, there is an increasing need to protect the user from accidental needle injury.

A catheter design which is directed toward this need is shown in U.S. Patent No. 4,762,516. The catheter shown in this application includes an elongate body which houses a sliding needle guard. As the needle is withdrawn from the emplaced catheter, the user pushes the tab at the distal end of the needle guard, thereby sliding the needle guard out of the housing and along the needle, until the distal end of the guard covers the needle tip and the proximal end of the guard locks in the housing. The needle and guard may then be set aside with the needle tip fully protected.

When an I.V. catheter is inserted into the patient, the user must have an indicator of some sort to signal successful entry of the introducer needle into the vein. The indicator is known as flashback in the flashchamber.

Upon successful entry, the blood must travel through the length of the hollow needle and into a clear or translucent chamber opposite the penetrating end of the needle. The appearance of blood in this chamber is the flashback, and the chamber itself is the flashchamber. Once this occurs, the user stops advancing the needle, and begins the process of threading the catheter tube into the vein.

It is important that the flashback appear quickly after entry into the vein, or the user risks the possibility of continuing the advancement of the needle through the vein, and out the other side, without knowing they were at one time already in the vein.

For a number of reasons, flashback can be delayed or even prevented completely. One reason is the resistance to the flow of blood through the needle caused by the inability of the blood to easily wet the internal surface of the needle. This is a result of the surface tension of the liquid and the surface energy of the stainless steel. This causes a resistance to flow that requires pressure to overcome.

In most cases, this surface tension resistance is not sufficient to stop the flow of blood through the needle upon entry into the vein, because the pressure of the blood overcomes all the resistance offered. In some cases, though, especially with the smaller needle sizes, such as in neonatal cases, the surface tension resistance is sufficient to impede, or stop the flow, becoming the predominant factor in the success or failure of the flashback indicator to perform properly.

### Summary of the Invention

Therefore, it is an object of the invention to reduce the surface tension resistance to blood flow through an intravenous catheter needle, or, in some instances to eliminate the resistance to blood flow.

It is further an object of the invention to increase the probability of instant flashback in the intravenous catheter device by reducing the surface tension resistance to blood flow in the needle.

These and other objects of the invention are accomplished in a catheter device containing a hollow introducer needle and a catheter which is inserted over the needle, and by providing for a surface tension reducer to be coated on the interior of the hollow introducer needle. In this manner, the surface tension is reduced and blood flows virtually instantaneously from the pierced vein to the flashback chamber. Therefore, the intravenous catheter user is able to immediately ascertain the piercing of the vein and properly position the intravenous catheter within the vein. Catheter use and function are enhanced, and the risk of even minor patient injury is greatly reduced.

These and other objects of the invention will be more better understood from the attached Detailed Description of the Drawings, taken in conjunction with the Detailed Description of the Invention which follows.

### Detailed Description of the Drawings.

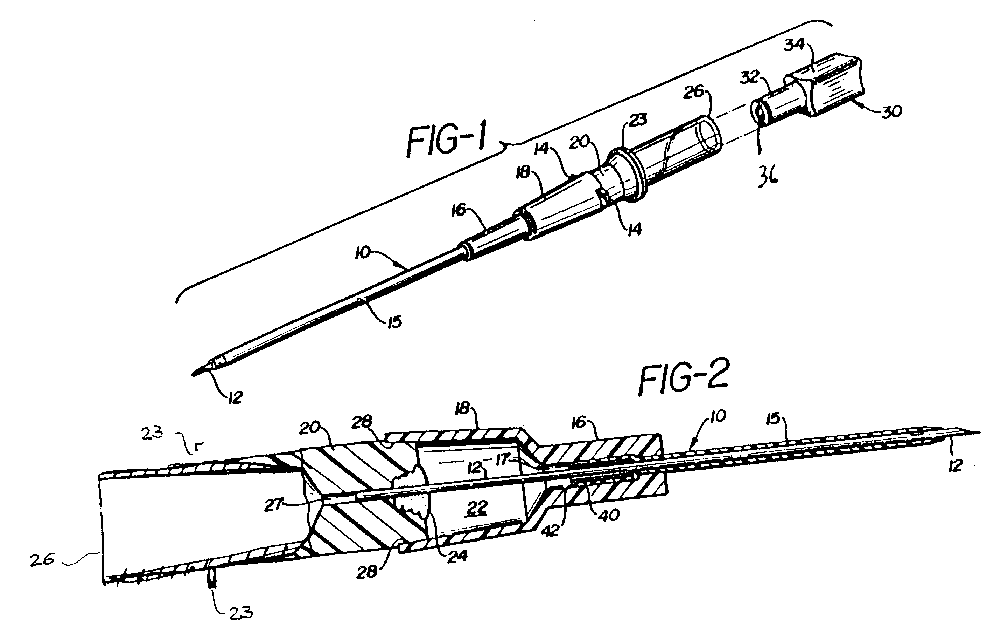

Figure 1 illustrates an intravenous catheter in perspective and constructed in accordance with the principles of the present invention; and Figure 2 illustrates the intravenous catheter of Fig. 1 in partial cross-section, without a needle guard.

### Detailed Description of the Invention.

Referring first to Fig. 1, an I.V. catheter constructed in accordance with the principles of the present invention is shown. The catheter 10 comprises a tube 15 made of fluorinated ethylene propylene or polyurethane material. The tube 15 is tapered at its distal end where it may easily slide into an opening in the patient's body formed by a hollow needle 12. The proximal end of catheter tube 15 is concentrically affixed within the distal end of a catheter hub 16, 18. The dual diameter hub terminates at its proximal end in a tab or fitting 14, suitable for attaching the catheter hub to a tubing set which administers a source of intravenous fluid.

The catheter 10 is engaged by the hollow needle 12, which is affixed at its proximal end to a needle hub 20. The needle hub 20 is hollow and includes a proximally located flange 23. Molded to the flange is a hollow flash chamber 26, which is made of a transparent or translucent polymer such as polypropylene or polycarbonate. The proximal end of the needle terminates in the hub 20, just short of the flash chamber.

Inserted into the flash chamber 26 (but shown separated just prior to insertion in Fig. 1) is a flash plug 30. The flash plug 30 is made of a polymeric material and will frictionally remain in place when inserted into the flash chamber. The flash plug body comprises a smaller diameter distal end 32 sized for a frictional fit in the flash chamber 26, and a somewhat square proximal end 34 with convex sides, which enables the flash plug to be easily grasped and inserted into the flash chamber. The flash plug 30 is hollow with an aperture 36 extending completely through the flash plug body. The distal opening of the aperture 36 is visible in Fig. 1. An insert made of a porous material is located within the aperture 36. The porous insert is pervious to the flow of air out of the flash chamber as the chamber fills with blood, but the pores of the insert are small enough to impede any blood flow therethrough.

Referring next to Fig. 2, the catheter 15 and its hub 16, 18 and the needle 12 and needle hub 20 are shown in cross-sectional detail. The proximal end of the catheter 15 is secured within passageway 17 of the distal end 16 of the catheter hub by a sleeve 40. The sleeve 40 is positioned within the proximal end of the lumen of the catheter 15. By forcing sleeve 40 into the lumen of the catheter, the sleeve effects an expansion of the proximal end of the catheter into contact with the internal surface of the passageway 17, thereby locking the hub and catheter together to prevent axial movement therebetween. The sleeve 40 is outwardly tapered at its proximal end to form a flange 42 which acts to securely position the sleeve 40 relative to the passageway 17.

The hollow needle 12 extends from its distal tip through the catheter 15 and sleeve 40, and into a passageway 27 in the center of the needle hub 20. The proximal portion of the needle 12 is affixed by epoxy 24 at the distal end of the needle hub 20. The proximal end of the needle 12 extends beyond the epoxy and into the passageway so that the lumen of the needle will not become filled with epoxy during the attachment procedure.

The distal end 28 of the needle hub 20 is of a lesser diameter than the major portion of the needle hub. This lesser diameter end is dimensioned to engage the open proximal end of the catheter hub 18.

In a conventional catheter and introducer needle assembly, the lesser diameter distal end of the needle hub is generally elongated and tapered to substantially occupy the proximal interior of the catheter hub. In the catheter assembly of the present invention, it may be seen that the major portion 22 of the cavity within the proximal end 18 of the catheter hub is unoccupied, with only the needle 12 extending through this region. This insures proper retention between needle hub 20 and catheter hub 18.

The ability of a liquid to wet the surface of a solid is determined by the relative energies of the liquid and the solid at their place of contact. The surface energy, or tension, of liquid is the force per unit length on the surface that opposes the expansion of the surface area. Solids also have surface tensions, but they are more difficult to measure. A common method of measuring the surface energy of a particular liquid in contact with a particular solid is to measure its contact angle or the angle formed at the liquid-solid interface of a liquid drop placed on the solid surface. A contact angle of zero indicates complete wetting and finite angles indicate varying degrees of incomplete wetting.

In the case of a stainless steel catheter needle 12 inserted into a blood vessel, the venous pressure pushes the blood into the needle 12. The speed at which the blood enters the tube and the distance the blood will travel up the tube depends on the venous pressure, the inner diameter of the needle 12, and the surface tensions of the needle 12 and the blood. If the liquid sufficiently wets the needle 12 walls, the level blood will flow is proportional to the liquid-solid tension and to the reciprocal of the needle 12 radius. The column of liquid inside the needle forms a curved surface or meniscus which represents its lowest possible energy state. The contraction of the liquid surface, due to surface tension, creates a pressure inside the liquid column which is higher than the atmospheric pressure pushing against it. Both this column pressure and the venous pressure allow blood to enter the needle until the pressure difference between the blood at its curved surface and the blood in the vein are balanced by the hydrostatic pressure of the blood column. The larger the inner diameter of the bore of needle 12, the shorter the distance blood will travel into the needle 12 at a constant venous pressure.

If the surface tension of the blood is sufficiently lower than the stainless steel, the blood will wet the needle surface and will rapidly flow through the needle. But if the surface tension of the blood is greater than that of the needle 12, the blood will not readily flow into the needle 12. Heparinized blood has a surface tension of about 56 x 10⁻⁵ N/cm (dynes/cm). This is somewhat lower than that of water at 72 x 10⁻⁵ N/cm (dynes/cm), but is still too high to allow rapid wetting of a stainless steel needle surface. Apparently, at this energy the force exerted on the blood, outside the needle 12, is not high enough to cause a capillary rise in the bore of the needle 12.

To overcome this large difference in surface energies between blood and stainless steel, compounds known as surface active agents or more commonly called surfactants, can be introduced to the system. The role of the surfactant is to lower the tension of the solid-liquid interface to achieve complete wetting. The surfactants are amphipathic molecules having one end which is relatively hydrophilic and one end which is relatively hydrophobic. When applied to the interior surface of needle 12, these compounds present a boundary layer which has a low surface tension as well as a low interfacial tension toward the blood. The lower energies allow the blood to readily spread over the stainless steel surface and result in a rapid filling of the cannula when the tip of the needle enters a patient's vein.

### Example 1

A 26 gauge hollow catheter needle was first tested without any surfactant applied to the inside. Surface tension, as indicated, was about 56 x 10⁻⁵ N/cm (dynes/cm) for heparinized blood within the needle. Then, the same needle was coated with a Pluronic™ polyol available from BASF Wyandotte Corporation, Wyandotte, Michigan 48192. These polyols are a series of closely related block polymers that may be generally classified as polyoxypropylene polyoxyethylene condensate, whose various grades fall into a molecular weight range between 1,000 and 15,000. In fact, the specific surfactant used was pluronic F68. The surface of the catheter needle was coated with the surfactant to achieve a uniform thin film around the entire inside of the needle. After the surfactant was allowed to dry onto the surface, blood was again tested. Flashback was virtuallly instanteous and surface tension was reduced to about less than 20 x 10⁻⁵ N/cm (dynes/cm).

Similar to the pluronic polyol used in the example, it is realized that many of the homologous series of nonylphenyloxypoly-(ethyleneoxy) ethanols may be available and useful as surfactants. Some of these such ethanols are available from GAF Corporation and are described as the Igepal™ CO non-ionic surfactants. Also available from GAF and useful as coating agents are the Igepal™ CA non-ionic surfactants. These are generally octylphenoxypoly-(ethyleneoxy) ethanols. Also, the Zonyl™ flurosurfactants available from DuPont Corporation, Wilmington, Delaware are also perceived as surfactants which interact well with the human body and yet also are able to improve surface tension within a catheter needle.

Therefore, it is realized that while many possible embodiments have been disclosed, it is to be realized that the invention described herein is to be understood from the attached claims.

## Claims

1. A catheter device (10) containing a hollow introducer needle (12) with cylindrical interior walls, and a catheter (15) which is inserted into the body over said needle, characterised by the interior of said needle having a surface tension reducer coated thereon.

2. A catheter device (10) comprising a hollow introducer needle (12) having open ends connected by interior cylindrical walls for insertion into a patient, said introducer needle supporting a tubular catheter (15), and a flash chamber (26) attached to said introducer needle at one of said open ends, characterised by said introducer needle having an interior surface treated to reduce surface tension to allow blood flow between said opposite open end and said flash chamber.

3. A catheter device according to claim 1 or claim 2, in which the surface tension reducer comprises a surfactant.

4. A catheter device according to claim 3, in which the surfactant comprises a pluronic polyol.

5. A catheter device according to claim 3, in which the surfactant comprises a nonylphenoxypoly-(ethyleneoxy) ethanol.

6. A catheter device according to claim 3, in which the surfactant comprises an octylphenoxypoly-(ethyleneoxy) ethanol.

7. A catheter device according to claim 3, in which the surfactant is a fluorinated surfactant.

## Patentansprüche

1. Kathetervorrichtung (10), enthaltend eine hohle Einführnadel (12) mit zylindrischen Innenwänden, und einen Katheter (15), der über diese Nadel in den Körper eingeführt wird, dadurch gekennzeichnet, daß das Innere der Nadel mit einem die Oberflächenspannung reduzierenden Mittel beschichtet ist.

2. Kathetervorrichtung (10), umfassend eine hohle Einführnadel (12) mit offenen Enden, die durch zylindrische Innnenwände verbunden sind, zum Einführen in einen Patienten, wobei die Einführnadel einen röhrenförmigen Katheter (15) trägt, und eine Flash-Kammer (26), die an einem der offenen Enden an der Einführnadel befestigt ist, dadurch gekennzeichnet, daß die Einführnadel eine innere Oberfläche aufweist, die behandelt wurde, um die Oberflächenspannung zu reduzieren, um das Fließen von Blut zwischen dem gegenüberliegenden offenen Ende und der Flash-Kammer zu ermöglichen.

3. Kathetervorrichtung nach Anspruch 1 oder Anspruch 2, wobei das die Oberflächenspannung reduzierende Mittel ein Surfactant umfaßt.

4. Kathetervorrichtung nach Anspruch 3, wobei das Surfactant ein Pluronic-Polyol umfaßt.

5. Kathetervorrichtung nach Anspruch 3, wobei das Surfactant ein Nonylphenoxypoly-(ethylenoxy)-ethanol umfaßt.

6. Kathetervorrichtung nach Anspruch 3, wobei das Surfactant ein Octylphenoxypoly-(ethylenoxy)-ethanol umfaßt.

7. Kathetervorrichtung nach Anspruch 3, wobei das Surfactant ein fluoriertes Surfactant ist.

## Revendications

1. Dispositif (10) formant cathéter contenant une aiguille creuse d'introduction (12) ayant des parois intérieures cylindriques, et un cathéter (15) qui est inséré à l'intérieur du corps par-dessus ladite aiguille, caractérisé en ce que la partie intérieure de ladite aiguille est revêtue d'un réducteur de tension superficielle.

2. Dispositif (10) formant cathéter comportant une aiguille creuse d'introduction (12) ayant des extrémités ouvertes reliées par des parois cylindriques intérieures pour être inséré à l'intérieur d'un patient, ladite aiguille d'introduction supportant un cathéter tubulaire (15), et une chambre de reflux (26) fixée sur ladite aiguille d'introduction au niveau de l'une desdites extrémités ouvertes, caractérisé en ce que ladite aiguille d'introduction a une surface intérieure traitée pour réduire la tension superficielle pour permettre l'écoulement du sang entre ladite extrémité ouverte opposée et ladite chambre de reflux.

3. Dispositif formant cathéter selon la revendication 1 ou 2, dans lequel le réducteur de tension superficielle est constitué d'un tensioactif.

4. Dispositif formant cathéter selon la revendication 3, dans lequel le tensioactif est constitué d'un polyol pluronic.

5. Dispositif formant cathéter selon la revendication 3, dans lequel le tensioactif est constitué d'un nonylphénoxypoly-(éthylèneoxy) éthanol.

6. Dispositif formant cathéter selon la revendication 3, dans lequel le tensioactif est constitué d'un octylphénoxypoly-(éthylèneoxy) éthanol.

7. Dispositif formant cathéter selon la revendication 3, dans lequel le tensioactif est un tensioactif fluoruré.
